(19) Europäisches Patentamt
European Patent Office
Office européen des brevets

(11) Veröffentlichungsnummer : **0 004 622**
**B1**

(12) # EUROPÄISCHE PATENTSCHRIFT

(45) Veröffentlichungstag der Patentschrift : **25.03.81**

(51) Int. Cl.$^3$ : **C 07 C 47/20**, C 07 C 45/00

(21) Anmeldenummer : **79100902.0**

(22) Anmeldetag : **24.03.79**

(54) **Verfahren zur Herstellung von Crotonaldehyd und Crotonaldehydderivaten.**

(30) Priorität : **11.04.78 DE 2815539**

(43) Veröffentlichungstag der Anmeldung : **17.10.79 (Patentblatt 79/21)**

(45) Bekanntmachung des Hinweises auf die Patenterteilung : **25.03.81 Patentblatt 81/12**

(84) Benannte Vertragsstaaten : **BE CH DE FR GB**

(56) Entgegenhaltungen : **Keine**
**JOURNAL OF THE AMERICAN CHEMICAL SOCIETY, volume 54, November 1932**
**Eaton**
**A.F. SHEPHARD et al. : « Rearrangement of unsaturated 1,4-Glycols 2-Methyl-2-butene-1,4-diol »**
**Seiten 4385-4391**

(73) Patentinhaber : **BASF Aktiengesellschaft**
**Carl-Bosch-Strasse 38**
**D-6700 Ludwigshafen (DE)**

(72) Erfinder : **Fischer, Rolf, Dr.**
**Bergstrasse 98**
**D-6900 Heidelberg (DE)**
Erfinder : **Weitz, Hans-Martin, Dr.**
**Auf dem Koeppel 40**
**D-6702 Bad Duerkheim 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 622 B1

Imprimerie Jouve, 17, rue du Louvre, 75001 Paris, France

Verfahren zur Herstellung von Crotonaldehyd und Crotonaldehydderivaten

Die Erfindung betrifft ein Verfahren zur Herstellung von Crotonaldehyden durch Umsetzung von 1,4-Diacyloxy-2-butenen mit Wasser in Gegenwart von Kationenaustauschern.

Es ist bekannt, daß man Tiglinaldehyd (2-Methylcrotonaldehyd) unter Kohlendioxid durch Kondensation von Acetaldehyd und Propionaldehyd in Gegenwart von Natronlauge in einer Ausbeute von 30 Prozent (Journal für praktische Chemie, Band 155, Seiten 315 bis 316 (1940)), durch Umsetzung von 3,4-Epoxy-3-methyl-1-buten mit Palladiumacetylacetonat und Triphenylphosphin in einer Ausbeute von 26 bis 60 Prozent (Agricultural Biological Chemistry, Band 39, Seiten 2 431 bis 2 432 (1975)) oder durch Isomerisierung von 2-Äthylacrolein mit Calciumchlorid und Destillation (Liebigs Annalen der Chemie, Band 494, Seite 273 (1932)) herstellen kann.

Eine Arbeit in Journal of the American Chemical Society, Band 54, Seite 4 390 (1932) zeigt, daß Tiglinaldehyd durch Erwärmen von 2-Methyl-2-1,4-diol-diacetat in einer Lösung von Chlorwasserstoff in Methylalkohol erhalten werden kann und beschreibt weitere Synthesen mit dem unveresterten Diol durch Umsetzung in Gegenwart von wasserfreiem Zinkchlorid und Destillation bzw. durch Erhitzen in Anwesenheit bzw. Abwesenheit von Chlorwasserstoff. Alle diese Umsetzungen wurden mit kleineren Mengen, z.B. von 4 Gramm Diol, durchgeführt. Es wird ausdrücklich darauf hingewiesen, daß man bei der Umsetzung von größeren Mengen, nämlich von 40 bis 50 Gramm-Mengen, vom unveresterten Diol ausgeht und dieses bei 0 °C mit wäßriger 11-n-Salzsäure während länger als 2 Stunden umsetzt. Dann wird Pyridin zugesetzt und erst jetzt das Gemisch vorsichtig erwärmt, die wäßrige Phase abgetrennt und destilliert, der abgetrennte, rohe Tiglinaldehyd in leicht saurer Lösungeiner Wasserdampfdestillation unterworfen, getrocknet und erneut fraktioniert. Man erhält eine Ausbeute von 65 Prozent. Diese Ausführungsform wird als die vorteilhafteste bei größeren Ansätzen angesehen. Nachteilig an dem Verfahren ist insbesondere, daß man vom Diol ausgeht und äquimolare Mengen Säure verwendet, die später mit Pyridin wieder neutralisiert werden müssen. Größere Mengen an Salz fallen an und sind nachteilig. Zusätzlich müssen anschließend Pyridinreste durch Wasserdampfdestillation entfernt werden.

Alle diese Verfahren sind im Hinblick auf einfachen und wirtschaftlichen Betrieb, Ausbeute, Raum-Zeit-Ausbeut, Aufarbeitung und Reinheit des Endstoffes unbefriedigend.

Es wurde nun gefunden, daß man Crotonaldehyde der Formel

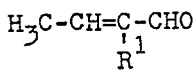 I,

worin $R^1$ ein Wasserstoffatom oder einen aliphatischen Rest bedeutet, vorteilhaft erhält, wenn man 1,4-Diacyloxy-2-butene der Formel

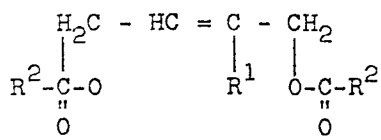 II,

worin $R^1$ die vorgenannte Bedeutung besitzt und $R^2$ ein Wasserstoffatom oder einen aliphatischen Rest bezeichnet, mit Wasser in Gegenwart eines Kationenaustauschers umsetzt.

Die Umsetzung kann im Falle der Verwendung von 2-Methyl-1,4-diacetoxy-2-buten durch die folgenden Formeln wiedergegeben werden :

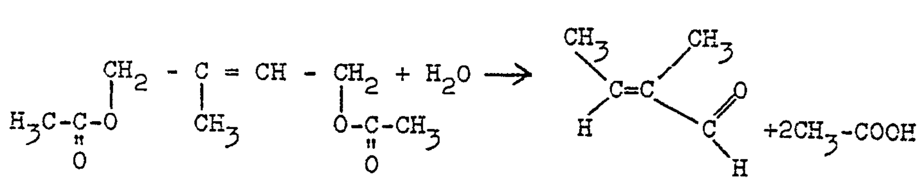

Im Vergleich mit den bekannten Verfahren liefert das Verfahren nach der Erfindung überrraschend auf einfacherem und wirtschaftlicherem Wege Crotonaldehyde und insbesondere Tiglinaldehyd in besserer Ausbeute und Reinheit. Die Aufarbeitung ist einfach, teuere oder schwer zugängliche Reaktionskomponenten sind nicht nötig. Vorteilhaft kann das Verfahren gerade in industriellem Maßstab durchgeführt werden. Der Ausgangsstoff kann in einstufiger Reaktion ohne vorangehende Umsetzung zum Diol zu Crotonaldehyden, insbesondere Tiglinaldehyd, umgesetzt werden. Große Mengen an Salz

infolge Neutralisation werden vermieden. Alle diese Vorteile des erfindungsgemäßen Verfahrens sind überraschend, denn es wäre gerade angesichts des Diesters als Ausgangsstoff im Hinblick auf den Stand der Technik eine wesentlich schlechtere Ausbeute zu erwarten gewesen.

Es war auch nicht vorauszusehen, wie 1,4-Diacetoxy-2-butene und insbesondere 2-Methyl-1,4-diacetoxy-2-buten und/oder die bei der Verseifung entstehenden entsprechenden Diole in Gegenwart von Wasser und Kationenaustauschern weiterreagieren würden. Nach Houben-Weyl, Methoden der Organischen Chemie, Band 6/3, Seiten 592 bis 594, bilden sich aus 1,4-Dihydroxyalkenen-(2) in Gegenwart sauer reagierender Katalysatoren Dihydrofurane. So setzt sich beispielsweise Buten-2-diol-1,4 mit Chlorwasserstoff oder Schwefelsäure zu 2,5-Dihydrofuran, Hexen-3-diol-2,5 mit Chlorwasserstoff zu 2,5-Dimethyl-2,5-dihydrofuran um. Die DE-A-20 62 950 beschreibt die Umsetzung von cis-2-Buten-1,4-diol-monoacetat oder-diacetat in Gegenwart von Säuren zu 2,5-Dihydrofuran.

Die Ausgangsstoffe, z.B. 2-Methyl-1,4-diacetoxy-2-buten, lassen sich, ausgehend z.B. von Isopren, entweder über Stoffe wie 2-Methyl-1,4-dibrom-2-buten und anschließende Umsetzung mit den Alkalisalzen entsprechender Carbonsäuren (Journal of the American Chemical Society, Band 54, Seite 4 388 (1932)) oder günstiger in einstufiger Reaktion durch Acyloxylierung von Olefinen, z.B. Acetoxylierung von Isopren mit Essigsäure und Sauerstoff in Gegenwart von tellurhaltigem Platin, entsprechend dem in der DE-A-24 17 658 beschriebenen Verfahren, herstellen. Bevorzugte Ausgangsstoffe II und dementsprechend bevorzugte Endstoffe I sind solche, in deren Formeln die einzelnen Reste $R^1$ und $R^2$ gleich oder verschieden sein können und jeweils ein Wasserstoffatom oder einen Alkylrest mit 1 bis 7 Kohlenstoffatomen bedeuten. Besonders bevorzugt bezeichnen $R^1$ und/oder $R^2$ einen Methylrest. Die vorgenannten Reste können noch durch unter den Reaktionsbedingungen inerte Gruppen, z.B. Alkylgruppen mit 1 bis 4 Kohlenstoffatomen, substituiert sein. Ebenfalls können als Ausgangsstoffe II solche Stoffe verwendet werden, die unter den Reaktionsbedingungen die Ausgangsstoffe II bilden, z.B. 1-Buten-3,4-diol-, 2-Methyl-1-buten-3,4-diol und 3-Methyl-1-buten-3,4-diol-diester. Es kommen z.B. folgende Ausgangsstoffe II in Betracht : Die Diester von 2-Buten-1,4-diol, 1-Buten-3,4-diol, 2-Methyl-2-buten-1,4-diol, 2-Methyl-1-buten-3,4-diol und 3-Methyl-1-buten-3,4-diol mit Ameisensäure, Propionsäure, Buttersäure, Isobuttersäure, Valeriansäure, Isovaleriansäure, Capronsäure, Önanthsäure und insbesondere Essigsäure ; homologe Diester des 2-Äthyl-, 2-Propyl-, 2-Isopropyl-, 2-Butyl-, 2-Isobutyl-, 2-sek.-Butyl-, 2-tert.-Butyl-, 2-Pentyl-, 2-Isopentyl-, 2-Hexyl-1,4-diols.

Die Umsetzung wird im allgemeinen bei Temperaturen von 0 bis 200 °C, vorzugsweise von 80 bis 120 °C, drucklos oder unter Druck, diskontinuierlich oder kontinuierlich in Gegenwart von Masser durchgeführt. Die Wassermenge beträgt vorteilhaft 1 bis 50, insbesondere 2 bis 20 Mol je Mol Ausgangsstoff I. Zusätzlich können unter den Reaktionsbedingungen inerte, organische Lösungsmittel verwendet werden. Hierfür kommen vor allem solche infrage, die mit Wasser mischbar sind oder ein gutes Lösungsvermögen für Wasser aufweisen, z.B. Alkanole und Cycloalkanole wie Äthanol, Methanol, n-Butanol, Isobutanol, tert.-Butanol, Glykol, Glycerin, sek.-Butanol, n-Propanol, Isopropanol.

Die Umsetzung wird in Gegenwart von sauren Ionenaustauschern, nämlich Kationenaustauschern, vorzugsweise sauren Kunstharzaustauschern, zweckmäßig in ihrer Säureform, durchgeführt. Solche Austauscher sind z.B. alle in Houben-Weyl, Methoden der Organischen Chemie, Band I/1, Seite 528, Tabelle 3 beschriebenen Kationenaustauscher. Vorzugsweise verwendet man stark und mittelstark saure Austauscher, z.B. Austauscher, die aus Styrol und Divinylbenzol aufgebaut sind und Sulfonsäuregruppen enthalten ; stark saure anorganische Kationenaustauscher wie Zeolithe ; Phenol- oder Polystyrolsulfonsäureharze, Styrolphosphonsäureharze, Styrolphosphinsäureharze, oder entsprechende saure Harze enthaltende Austauscher, z.B. bifunktionelle Kondensationsharze. So kommen z.B. vorgenannte Harze, die im Handel unter der Bezeichnung (R) Lewatit S 100, Amberlit IR-120, Lewasorb, Dowex 50 WX 8, Amberlyst 15 erhältlich sind, in Betracht. Die Menge der verwendeten Ausgangsstoffe bzw. des Austauschers hängt von der Selektivität bzw. der Zahl der austauschaktiven Gruppen der verwendeten Austauscher bei der Reaktionstemperatur ab, im allgemeinen verwendet man 1 bis 40 Gewichtsprozent, vorzugsweise 5 bis 25 Gewichtsprozent Austauscher, bezogen auf den Ausgangsstoff II. Die im trockenen Austauscher enthaltene Wassermenge, die je nach Struktur bis zu 50 Gewichtsprozent betragen kann, ist in dem oben definierten Zusatz an Wasser nicht einbegriffen. Form und Korngröße des Austauschers können in einem weiten Bereich beliebig gewählt werden. Bezüglich Herstellung und Einzelheiten der Verwendung von Ionenaustauschern wird auf das Kapitel « Ionenaustauscher » des genannten Werkes verwiesen.

Die Reaktion kann wie folgt durchgeführt werden : Ein Gemisch aus Ausgangsstoff II, Wasser, saurem Kationenaustauscher und gegebenenfalls organischem Lösungsmittel wird 0,5 bis 24 Stunden bei der Reaktionstemperatur gehalten. Dann wird der Endstoff in üblicher Weise, z.B. durch Filtration und fraktionierte Destillation des Filtrats, abgetrennt. Man kann auch, z.B. im Falle des Tiglinaldehyds, das Filtrat nach Zugabe von zusätzlichem Wasser destillieren, wobei bei Atmosphärendruck zwischen etwa 80 und 100 °C ein zweiphasiges Gemisch aus Tiglinaldehyd und Wasser, zwischen etwa 100 und 188 °C ein einphasiges Gemisch aus überwiegend Wasser und Essigsäure übergeht. Nach der Phasentrennung von Tiglinaldehyd und Wasser kann der Tiglinaldehyd, der schon eine hohe Reinheit besitzt, gegebenenfalls mit einem Trockenmittel getrocknet und erneut fraktioniert destilliert werden. Spuren an Carbonsäuren, z.B. Essigsäure, können durch Neutralisation entfernt werden. Besonders einfach kann der Tiglinaldehyd durch Abdestillieren und Kondensieren eines Tiglinaldehyd-Wassergemisches in einer

Vorrichtung zur kontinuierlichen Abnahme der oberen Destillatphase eines zweiphasigen Gemisches abgetrennt werden.

Die nach dem Verfahren der Erfindung erhältlichen Crotonaldehyde sind wertvolle Ausgangsstoffe für die Herstellung von Farbstoffen, Pflanzenschutzmitteln, Polymerisations-inhibitoren und Vulkanisationsbeschleunigern. Tiglinaldehyd kann z.B. als Zwischenprodukt für die Herstellung von Terpenen verwendet werden oder dient als Polymerisations-inhibitor bei der Herstellung von Polyvinylchlorid (US-A-2 616 887). Das syn.-Oxim des Tiglinaldehyds wird als Süßstoff in Lebensmitteln in der US-A-3 780 194 beschrieben. Bezüglich der Verwendung wird weiterhin auf die vorgenannten Veröffentlichungen und Ullmanns Encyklopädie der technischen Chemie, Band 5, Seite 616, verwiesen.

Die in den folgenden Beispielen angegebenen Teile sind Gewichtsteile ; sie verhalten sich zu den Raumteilen wie Kilogramm zu liter.

## Beispiel 1

a) In einem Reaktor werden nach dem Spülen der Apparatur mit Stickstoff 600 Teile Eisessig und 15 Teile eines Palladium und Tellur enthaltenden Katalysators (7,10 Gewichtsprozent Palladium und 1,9 Gewichtsprozent Tellur auf Aktivkohle) auf 95 °C erhitzt. Im Verlauf von 4 Stunden werden gleichzeitig 12 000 Volumenteile Sauerstoff eingeleitet und 36,5 Gewichtsteile Isopren zugegeben. Dann Leitet man nach beendeter Zugabe bei 95 °C 30 Minuten lang Stickstoff durch das Reaktionsgemisch, läßt abkühlen und entfernt den Katalysator durch Filtrieren oder Zentrifugieren. Das Filtrat wird fraktioniert destilliert. Man erhält 61,8 Teile 2-Methyl-1,4-diacetoxy-2-buten vom Siedepunkt 57 bis 59 °C/0,3 mbar, $n_D^{20}$ = 1,4478.

b) Es wird ein unter der Bezeichnung (R)Dowex 50 WX 8 (50 bis 100 mesh) im Handel erhältlicher Polystyrolsulfonsäureharz-Kationenaustauscher verwendet. Er wird mit Salzsäure vorbehandelt und mit Wasser neutral gewaschen. Ein Gemisch aus 100 Teilen 2-Methyl-1,4-diacetoxy-2-buten, 97 Teilen Wasser und 27 Teilen Kationenaustauscher wird eine Stunde lang unter Rückfluß gerührt. Nach dem Erkalten wird der Ionenaustauscher abgesaugt und das homogene Filtrat bei Normaldruck fraktioniert destilliert. Man erhält 38,3 Teile Tiglinaldehyd (90 % der Theorie, bezogen auf eingesetztes 2-Methyl-1,4-diacetoxy-2-buten) vom Siedepunkt 116,5 bis 118 °C/1 013 mbar ; $n_D^{20}$ = 1,4429.

## Beispiel 2

Es wird ein unter der Bezeichnung (R)Lewatit SPC 118 H im Handel erhältlicher Polystyrolsulfonsäureharz-Kationenaustauscher verwendet. Ein Gemisch aus 100 Teilen 2-Methyl-1,4-diacetoxy-2-buten, 97 Teilen Wasser und 27 Teilen des Kationenaustauschers wird, wie in Beispiel 1 beschrieben, umgesetzt und aufgearbeitet. Dabei werden 38,2 Teile Tiglinaldehyd (84,6 % der Theorie) vom Siedepunkt 117 bis 118 °C erhalten.

## Beispiel 3

Es wird ein unter der Bezeichnung (R)Dowex 50 WX 8 im Handel erhältlicher Polystyrolsulfonsäureharz-Kationenaustauscher verwendet. Ein Gemisch aus 100 Teilen 2-Methyl-1,4-diacetoxy-2-buten, 100 Teilen Wasser und 10,8 Teilen des Kationenaustauschers wird 4 Stunden lang unter Rückfluß gerührt und, wie in Beispiel 1 beschrieben, umgesetzt und aufgearbeitet. Dabei werden 39,2 Teile Tiglinaldehyd (86,8 % der Theorie) vom Siedepunkt 116 bis 119 °C erhalten.

## Beispiel 4

Es wird ein unter der Bezeichnung (R)Amberlite IR-120 (20 bis 50 mesh, Wassergehalt 44 bis 48 Prozent) im Handel erhältlicher Polystyrolsulfonsäureharz-Kationenaustauscher verwendet.

Ein Gemisch aus 119 Teilen 2-Methyl-1,4-diacetoxy-2-buten, 140 Teilen Wasser und 20 Teilen des wasserhaltigen Kationenaustauschers wird 2,5 Stunden lang auf 95 bis 100 °C erhitzt, wobei gleichzeitig der Tiglinaldehyd durch Abdestillieren und Kondensieren eines Tiglinaldehyd-Wassergemisches in einer Vorrichtung zur kontinuierlichen Abnahme der oberen Destillatphase eines zweiphasigen Gemisches abgetrennt wird. Nach dem Trocknen mit Magnesiumsulfat wird der Tiglinaldehyd mit 3 Teilen Natriumcarbonat versetzt. Man erhält nach Filtration und anschließender Destillation 46,2 Teile Tiglinaldehyd (85,9 % der Theorie) vom Siedepunkt 116 bis 119 °C, $n_D^{20}$ = 1,4455.

**Anspruch**

Verfahren zur Herstellung von Crotonaldehyden der Formel

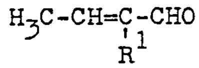 I,

worin $R^1$ ein Wasserstoffatom oder einen aliphatischen Rest bedeutet, *dadurch gekennzeichnet,* daß man 1-4-Diacyloxy-2-butene der Formel

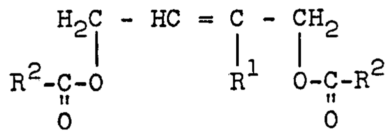 II,

worin $R^1$ die vorgenannte Bedeutung besitzt und $R^2$ ein Wasserstoffatom oder einen aliphatischen Rest bezeichnet, mit Wasser in Gegenwart eines Kationenaustauschers umsetzt.

**Revendication**

Procédé de préparation d'aldéhydes crotoniques de la formule

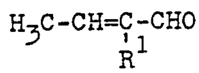 I,

dans laquelle $R^1$ désigne un atome d'hydrogène ou un reste aliphatique, caractérisé en ce que l'on fait réagir des 1,4-diacyloxy-2-butènes de la formule

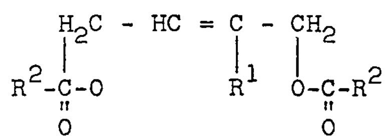 (II),

dans laquelle $R^1$ possède l'une des significations définies et $R^2$ désigne un atome d'hydrogène ou un reste aliphatique, avec de l'eau en présence d'un échangeur de cations.

**Claim**

A process for the preparation of a crotonaldehyde of the formula

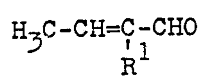 I,

where $R^1$ is hydrogen or an aliphatic radical, *characterized in that* a 1,4-diacyloxy-but-2-ene of the formula

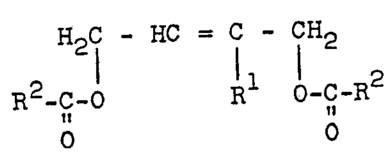 II,

where $R^1$ has the above meaning and $R^2$ is hydrogen or an aliphatic radical, is reacted with water in the presence of a cation exchanger.